(19) Europäisches Patentamt
European Patent Office
Office européen des brevets

(11) Numéro de publication: **0 177 431**
**B1**

(12) # FASCICULE DE BREVET EUROPEEN

(45) Date de publication du fascicule du brevet: **09.12.87**

(51) Int. Cl.⁴: **C 07 C 69/63**, C 07 C 67/287

(21) Numéro de dépôt: **85420112.6**

(22) Date de dépôt: **21.06.85**

(54) **Procédé de préparation de monocarboxylates de monohalogénohydroquinone et leur utilisation pour la préparation de dicarboxylates de monohalogénohydroquinone.**

(30) Priorité: **25.06.84 FR 8410183**

(43) Date de publication de la demande: **09.04.86 Bulletin 86/15**

(45) Mention de la délivrance du brevet: **09.12.87 Bulletin 87/50**

(84) Etats contractants désignés: **AT BE CH DE GB IT LI LU NL SE**

(56) Documents cités: **US - A - 2 588 978**

(73) Titulaire: **RHONE-POULENC CHIMIE, 25, quai Paul Doumer, F-92408 Courbevoie Cédex (FR)**

(72) Inventeur: **Ratton, Serge, Hameau de Belmont Vaulx Milieu, F-38090 Villefontaine (FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex (FR)**

**EP 0 177 431 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention se rapporte à un procédé de préparation de monocarboxylates de monohalogénohydroquinone à partir de monocarboxylates d'hydroquinone (plus particulièrement de monocarboxylates de monochlorohydroquinone à partir de monocarboxylates d'hydroquinone), et à leur utilisation pour la préparation de dicarboxylates de monohalogénohydroquinone et en particulier de dicarboxylates de monochlorohydroquinone.

Les dicarboxylates de monohalogénohydroquinone et en particulier les dicarboxylates de monochlorhydroquinone sont des produits industriels importants utilisés notamment pour la préparation de polyesters aromatiques (cf. brevet américain N° 4.118.372 et demande de brevet français N° 79-24.135 publiée sous le N° 2.465.758) ou comme produits intermédiaires pour la préparation des monohalogénohydroquinones, et en particulier de la monochlorhydroquinone, utilisées comme développeurs photographiques (cf. brevets américains N° 2.748.173 et N° 1.912.744).

Il existe différentes voies d'accès aux dicarboxylates de monochlorohydroquinone qui se distinguent les unes des autres par la nature des produits de départ. Ainsi, on a proposé de préparer le diacétate de monochlorohydroquinone à partir de la benzoquinone. Selon une première voie d'accès, on procède à l'acétylation réductrice de la chlorobenzoquinone [cf. J. CASON et al., J. Org. Chem. *15* 860-4 (1950)]. Selon une seconde voie d'accès, on procède simultanément à la chloration et à l'acétylation réductrice de la benzoquinone par réaction de cette dernière soit avec le chlorure d'acétyle, éventuellement en présence de zinc [cf.: SCHIED Annalen *218*, page 213 (1883); G.A. VARVOGLIS Ber. *70* page 2396 (1937); H. BURTON et al., J. Chem. Soc. *1952* pages 2546-48], soit avec l'anhydride acétique en présence de chlorure de zinc [cf.: THIELE et al., Annalen *311* page 344 (1900); H. BURTON et al., J. Chem. Soc. *1952* pages 755-59]. Ces divers procédés ne présentent pas d'intérêt industriel d'une part en raison de leur faible sélectivité (ils conduisent à la formation concomitante de quantités importantes de dérivés dichlorés et de diacétate d'hydroquinone), d'autre part du fait même de l'utilisation de la benzoquinone comme produit de départ. C'est la raison pour laquelle le procédé le plus intéressant industriellement consiste à préparer le diacétate de monochlorohydroquinone à partir d'hydroquinone, produit industriel courant. Ce procédé comprend une étape de chloration de l'hydroquinone en monochlorohydroquinone suivie d'une étape d'acétylation de la monochlorhydroquinone. Cette dernière étape ne pose pas de problème particulier car les rendements en diacétate de monochlorohydroquinone sont quantitatifs; on utilise comme agent d'acétylation le chlorure d'acétyle [SCHIED, loc. cit.] ou l'anhydride acétique [cf. LEVY et al., Annalen *210* page 140 (1881); VAN HERP Ber. *58* page 664 (1925)]. Par contre la mise en œuvre de l'étape de chloration se révèle plus délicate et c'est de son déroulement que dépend la qualité du produit final. En effet les divers procédés de chloration de l'hydroquinone qui ont été proposés impliquent la formation concomitante de quantités importantes de dichlorohydroquinones difficiles à séparer de la monochlorohydroquinone et qui conduisent à la formation de diacétates de dichlorohydroquinone après acétylation, eux aussi difficiles à séparer du diacétate de monochlorohydroquinone. Le problème de la chloration sélective de l'hydroquinone en monochlorohydroquinone est ancien (cf. brevet américain N° 1.912.744) et n'a pu être résolu de façon entièrement satisfaisante. Dans le brevet américain 2.748.173 on a proposé de réaliser la chloration de l'hydroquinone par réaction du chlore gazeux avec une solution d'hydroquinone dans l'acide acétique aqueux; en dépit des précautions prises (mise en œuvre d'un défaut de chlore qui se traduit par un taux de transformation incomplet de l'hydroquinone), la formation de dichlorohydroquinones reste importante. Dans la demande de brevet japonais publiée sous le N° 56/45.433, on a proposé de résoudre ce problème en procédant à la chloration à l'aide d'une solution aqueuse 5N d'acide chlorhydrique en présence de chlorures ferrique ou cuivrique et d'un gaz contenant de l'oxygène sous pression. Bien que ce procédé permette de limiter la formation des dichlorohydroquinones, son intérêt industriel est diminué par la nécessité d'opérer sous pression d'oxygène et de recourir à la présence de sels métalliques qui favorisent l'oxydation de l'hydroquinone en quinone.

Dès lors l'industrie est toujours à la recherche d'un procédé permettant d'accéder de manière sélective aux dicarboxylates de monohalogénohydroquinone et plus particulièrement aux dicarboxylates de monochlorohydroquinone. C'est ce problème que se propose précisément de résoudre la présente invention en mettant à la disposition de l'industrie un procédé sélectif d'obtention de monocarboxylates de monohalogénohydroquinone utilisables pour préparer des dicarboxylates de monohalogénohydroquinone et notamment des dicarboxylates de monochlorohydroquinone ou pour obtenir éventuellement des monohalogénohydroquinones.

Un premier objet de l'invention réside donc dans un procédé de préparation sélectif de monocarboxylates de monohalogénohydroquinone.

Un autre objet de l'invention réside également dans l'utilisation des monocarboxylates de monohalogénohydroquinone ainsi obtenus pour la préparation de dicarboxylates de monohalogénohydroquinone.

Plus spécifiquement un premier objet de l'invention réside dans un procédé de préparation de monocarboxylates de monohalogénohydroquinone, caractérisé en ce que l'on procède à l'halogénation d'un monocarboxylate d'hydroquinone éventuellement en mélange avec un dicarboxylate d'hydroquinone et une quantité mineure d'hydroquinone au moyen d'un agent halogénant pris dans le groupe formé par le chlore, le brome ou les halogénures de sulfuryle dans un acide carboxylique sensiblement anhydre.

On a constaté en effet que les monocarboxylates d'hydroquinone peuvent être halogénés très sélectivement dans ces conditions à la différence de l'hydroquinone. Les monocarboxylates de monohalogénohydroquinone ainsi obtenus peuvent être transformés aisément dans le milieu d'halogénation en dicarbocylates de monohalogénohydroquinone par acylation. L'enchaînement de ces deux étapes constitue donc un moyen commode et sélectif de préparation de dicarboxylates de monohalogénohydroquinone.

Le procédé de l'invention convient tout particulièrement bien à la préparation de monocarboxylates de monohalogénohydroquinone et de dicarboxylates de monohalogénohydroquinone de formules générales:

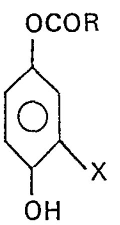
et
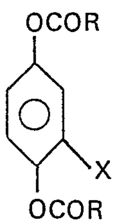

(I) (II)

dans lesquelles:

R représente un radical alkyle linéaire ou ramifié ayant de 1 à 4 atomes de carbone,

X représente un atome de chlore ou de brome, à partir de monocarboxylates d'hydroquinone de formule générale:

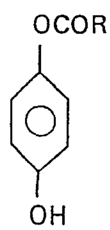
(III)

dans laquelle R a la signification donnée ci-avant.

Comme exemples de radicaux R on peut citer les radicaux méthyle, éthyle, n-propyle, isopropyle, n-butyle, isobutyle.

Comme exemples de monocarboxylates d'hydroquinone justiciables du présent procédé, on peut citer spécifiquement les monoacétate, propionate, n-butyrate et isobutyrate d'hydroquinone.

D'une manière générale l'halogénation des monocarboxylates d'hydroquinone peut être réalisée dans les conditions utilisées habituellement pour substituer un atome d'halogène à un atome d'hydrogène d'un noyau aromatique au moyen de chlore, de brome ou d'halogénure de sulfuryle.

La quantité d'agent halogénant est en général voisine de la stœchiométrie de la réaction, c'est-à-dire voisine de la quantité théoriquement nécessaire pour introduire un atome d'halogène par mole de monocarboxylate d'hydroquinone. On ne sortirait pas du cadre de la présente invention en mettant en œuvre un léger défaut ou un excès d'agent halogénant. Sur un plan pratique une quantité d'agent d'halogénation comprise dans un intervalle de 0,5 à 1,5 mole par mole de monocarboxylate d'hydroquinone convient bien.

La température à laquelle est conduite la réaction d'halogénation peut varier dans de larges limites; en général elle est choisie dans un intervalle de 0 à 120° C et de préférence de 10 à 100° C.

Comme acides carboxyliques on utilise de préférence des acides bons solvants des monocarboxylates d'hydroquinone, des dicarboxylates d'hydroquinone et de l'hydroquinone, stables et liquides dans les conditions de la réaction. Par acides sensiblement anhydres on désigne des acides contenant moins de 5% en poids d'eau et de préférence moins de 2% en poids. On a plus particulièrement recours à des acides alcanoïques, linéaires ou ramifiés comportant de 1 à 8 atomes de carbone tels que les acides formique, acétique, propionique, butyrique, isobutyrique, méthyl-2 butanoïque, éthyl-2 butanoïque, diméthyl-2,2 butanoïque, pentanoïque, méthyl-2 pentanoïque, méthyl-5 pentanoïque, hexanoïque éthyl-2 hexanoïque. Pour des raisons de commodité on a recours préférentiellement aux acides alcanoïques inférieurs ayant de 1 à 4 atomes de carbone. De préférence on utilise l'acide carboxylique dont dérive le monocarboxylate d'hydroquinone.

La concentration du monocarboxylate d'hydroquinone dans l'acide carboxylique n'est pas critique et peut varier dans de larges limites. Son choix dépend essentiellement de considérations d'ordre pratique liées à l'obtention d'un compromis entre une productivité aussi élevée que possible de l'appareillage et une bonne agitabilité du mélange réactionnel.

Les monocarboxylates d'hydroquinone peuvent être mis en œuvre soit seuls, soit en mélange avec des dicarboxylates d'hydroquinone et éventuellement des quantités mineures d'hydroquinone. Comme il n'est pas aisé d'obtenir les monocarboxylates d'hydroquinone exempts des dicarboxylates [pour cela il faut avoir recours à des méthodes de préparation compliquées telles que celle décrite par H.S. OLCOTT, J. Am. Chem. Soc. *59* pages 392-393 (1937)], on préfère mettre en œuvre le procédé de l'invention en faisant appel à des mélanges de monocarboxylates d'hydroquinone, de dicarboxylates d'hydroquinone et éventuellement d'hydroquinone obtenus par acylation partielle de l'hydroquinone au moyen d'agents d'acylation tels que les anhydrides et les halogénures d'acides [cf. F.D. CHATTAWAY, J. Chem. Soc. *1931* pages 2495-96; D. JOHNSTON, Chem. and Ind. *1982* page 1000] ou encore par réaction de redistribution entre l'hydroquinone et les dicarboxylates d'hydroquinone. L'utilisation de tels mélanges se révèle particulièrement avantageuse car on a constaté que lors de l'halogénation les dicarboxylates d'hydroquinone ne sont pratiquement pas halogénés et ne donnent par conséquent pas naissance à des dérivés dihalogénés. La présence d'une faible quantité d'hydroquinone n'est pas non plus préjudiciable à la sélectivité de l'halogénation. En fin de réaction le monocarboxylate de monohalogénohydroquinone est séparé des autres constituants du mélange. Lorsque la masse réactionnelle d'halogénation est soumise à l'acylation, la monohalogénohydroquinone éventuellement formée au cours de l'halogénation (et qui peut être accompagnée de très faibles quantités de dihalogénohydroquinones) est transformée en dicarboxylate de monohalogénohydroquinone; le dicarboxylate de monohalogénohydroquinone est alors séparé de l'ensemble du dicarboxylate d'hydroquinone qui peut être réutilisé pour la préparation du mélange de monocarboxylate d'hydroquinone et de dicarboxylate d'hydroquinone.

Le recours à des mélanges de monocarboxylate d'hydroquinone et de dicarboxylate d'hydroquinone contenant des quantités mineures d'hydroquinone pour mettre en œuvre le procédé de l'invention se révèle donc particulièrement avantageux et constitue de ce fait un autre objet de la présente invention. L'utilisation de mélange de monocarboxylates d'hydroquinone, de dicarboxylates d'hydroquinone et d'hydroquinone provenant de la réaction de redistribution d'un dicarboxylate d'hydroquinone avec l'hydroquinone représente un mode préféré de mise en œuvre du procédé de préparation des monocarboxylates de monohalogénohydroquinone selon l'invention. Cette réaction peut être représentée par le schéma réactionnel suivant:

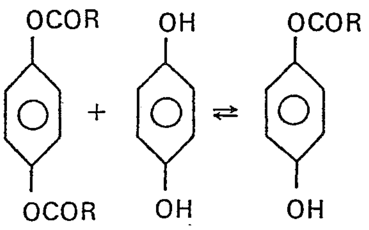

(1)

dans lequel R a la signification donnée plus haut.

Pour mener à bien cette réaction, on met en contact des quantités de dicarboxylates d'hydroquinone et d'hydroquinone calculées pour déplacer le plus possible vers la droite l'équilibre représenté par le schéma (1). Afin de limiter le plus possible la quantité d'hydroquinone présente dans le mélange soumis à l'halogénation et, par conséquent, la formation des dihalogénohydroquinones, on opère en présence d'un excès de dicarboxylate d'hydroquinone puisque ce dernier est pratiquement inerte lors de l'halogénation. L'excès de dicarboxylate d'hydroquinone nécessaire à l'obtention d'un produit final contenant aussi peu de dérivés dihalogénés que possible dépend de la pureté du produit final désiré et des conditions des réactions de redistribution et d'halogénation. D'une manière générale il est préférable de mettre en œuvre un excès d'au moins 0,5 mole de dicarboxylate d'hydroquinone par rapport à la stœchiométrie de la réaction de redistribution; il n'y a pas de limite supérieure critique à l'excès de dicarboxylate d'hydroquinone; cependant l'emploi d'une quantité trop importante de dicarboxylate d'hydroquinone entraînerait une baisse de la productivité de la réaction et des frais élevés de séparation des dicarboxylates d'hydroquinone; pour ces raisons il n'est pas nécessaire que l'excès de dicarboxylate d'hydroquinone soit supérieur à 4 moles par rapport à la quantité stœchiométrique. En résumé la quantité de dicarboxylate d'hydroquinone mise en œuvre à la phase de redistribution est d'au moins 1,5 mole par mole d'hydroquinone; elle est de préférence comprise dans un intervalle de 1,5 à 5 moles par mole d'hydroquinone.

La réaction de redistribution peut être conduite en masse ou dans tout milieu organique inerte dans les conditions de la réaction. On fait appel de préférence à des solvants des réactifs mis en œuvre. Les acides carboxyliques liquides dans les conditions de la réaction, les éthers aliphatiques ou arylaliphatiques tels que l'oxyde d'isopropyle, l'oxyde de dibutyle, l'anisol; les ethers éthérocycliques tels que le tétrahydrofuranne, le dioxanne; des hydrocarbures aliphatiques saturés (n-hexane), cycloaliphatiques (cyclohexane), aromatiques (benzène, toluène) ou leurs dérivés halogénés: tétrachlorure de carbone, chlorure de méthylène, chloroforme, chlorobenzène conviennent tout particulièrement bien. Les mêmes acides carboxyliques que ceux cités pour la phase d'halogénation peuvent être utilisés. On a recours plus particulièrement aux acides alcanoïques inférieurs et notamment à l'acide acétique, de préférence anhydres. La concentration des réactifs dans le milieu réactionnel n'est pas critique.

La température de la réaction de redistribution peut varier dans de larges limites. En général des températures comprises dans un intervalle de 50 à 250° C et de préférence de 80 à 180° C conviennent bien. La réaction peut être conduite à pression normale ou sous pression; lorsque la température choisie est supérieure au point d'ébullition de certains des constituants du mélange, on peut opérer sous la pression autogène des réactifs.

Lorsqu'on conduit la redistribution dans un acide carboxylique, on peut opérer en présence ou en absence de catalyseur. Lorsqu'on utilise un catalyseur, on fait appel à des acides minéraux ou organiques forts, c'est-à-dire des acides présentant un pK dans l'eau à 25° C inférieur à 1. On fait appel de préférence à l'acide sulfurique et aux acides sulfoniques tels que les acides méthanesulfonique, di- et trifluorométhanesulfonique, benzènesulfonique, toluènesulfonique, naphtalènesulfonique ou aux résines sulfoniques.

La quantité d'acide fort exprimée en équivalent de protons par mole d'hydroquinone peut varier dans de larges limites. En général elle est comprise entre 0,0001 et 0,2 équivalent de proton par mole d'hydroquinone.

Lorsqu'on utilise un solvant autre qu'un acide et en particulier un éther, on peut opérer en présence d'un catalyseur du type de ceux qui sont utilisés dans les réactions de transestérification. A cet effet on peut faire appel à des bases azotées organiques telles que les amines primaires, secondaires ou tertiaires et les bases hétérocycliques; on peut citer plus particulièrement la diéthylamine, l'éthylamine, la triéthylamine, la n-propylamine, la diisopropylamine, la triéthanolamine, la pyridine, la pi-

péridine. On peut également faire appel à des carboxylates alcalins tels que les acétates de K, Li ou Na et à des acides de Lewis tels que ceux cités dans l'ouvrage de G.A. OLAH, Friedel-Crafts Reaction volume 1 pages 191 à 291. On fait appel de préférence à des sels de zinc, de titane, de manganèse, de cobalt ou à des alkoxydes métalliques. On peut citer notamment des halogénures de zinc tels que $ZnCl_2$; des titanates d'alkyle tels que les titanates de méthyle, d'éthyle, de n-propyle, d'isopropyle, de n-butyle, d'isobutyle; des carboxylates de Mn et de Co tels que les acétate, propionate et isobutyrate de Mn et de Co.

La quantité de catalyseur exprimée en moles de base ou d'acide de Lewis par mole d'hydroquinone peut être comprise dans un intervalle de 0,0001 à 0,2 mole par mole d'hydroquinone.

De préférence la réaction de redistribution est conduite dans un acide carboxylique et plus préférentiellement encore dans le même acide carboxylique que celui utilisé à la phase d'halogénation du mélange, l'acide fort éventuellement utilisé comme catalyseur n'étant d'aucune gêne pour le déroulement de l'halogénation. On fait appel de préférence à l'acide correspondant à celui dont dérivent les carboxylates mis en œuvre. Il est alors particulièrement avantageux d'enchaîner l'étape de redistribution et l'étape d'halogénation.

Un autre objet de la présente invention réside donc plus particulièrement dans un procédé de préparation de monocarboxylates de monohalogénohydroquinone suivant lequel on prépare dans une première étape un mélange de monocarboxylates d'hydroquinone, de dicarboxylates d'hydroquinone et d'une quantité mineure d'hydroquinone par réaction d'hydroquinone avec un excès de dicarboxylates d'hydroquinone dans un acide carboxylique sensiblement anhydre éventuellement en présence d'un acide fort puis, dans une deuxième étape, on soumet le mélange réactionnel de redistribution à une halogénation à l'aide de chlore, de brome ou d'un halogénure de sulfuryle.

Sur un plan pratique l'halogénation est réalisée de façon simple par addition progressive de l'agent d'halogénation à la solution carboxylique de monocarboxylate d'hydroquinone et éventuellement de dicarboxylate d'hydroquinone et d'hydroquinone maintenue à température convenable. En fin de réaction les différents constituants du milieu réactionnel peuvent être séparés par exemple par distillation ou bien, selon un autre mode de réalisation préféré de l'invention, traités par un agent acylant pour transformer le monocarboxylate de monohalogénohydroquinone.

Plus spécifiquement la présente invention a encore pour objet un procédé de préparation des dicarboxylates de monohalogénohydroquinone selon lequel on procède dans une première étape à l'halogénation, au moyen d'un agent d'halogénation pris dans le groupe formé par le chlore, le brome, le chlorure de sulfuryle dans un acide carboxylique sensiblement anhydre, d'un monocarboxylate d'hydroquinone ou d'un mélange de monocarboxylates d'hydroquinone, de dicarboxylates d'hydroquinone et d'une quantité mineure d'hydroquinone, en monocarboxylate de monohalogénohydroquinone, puis, dans une deuxième étape, à l'acylation du monocarboxylate d'hydroquinone au moyen d'un agent d'acylation éventuellement en présence d'un acide fort.

La similitude des milieux de réaction permet donc d'enchaîner les étapes d'halogénation et d'acylation sans séparer les produits d'halogénation.

De la même façon on peut enchaîner les étapes de préparation des mélanges de monocarboxylates d'hydroquinone, dicarboxylates d'hydroquinone et d'hydroquinone, en particulier par redistribution, et les étapes d'halogénation et d'acylation.

La présente invention a donc encore pour objet un procédé de préparation de dicarboxylates de monohalogénohydroquinone selon lequel on prépare dans une première étape un mélange de monocarboxylate d'hydroquinone, de dicarboxylate d'hydroquinone et d'une quantité mineure d'hydroquinone par réaction d'hydroquinone avec un excès de dicarboxylates d'hydroquinone dans un acide carboxylique sensiblement anhydre éventuellement en présence d'un acide fort puis, dans une deuxième étape, on soumet le mélange réactionnel de redistribution à une halogénation à l'aide de chlore, de brome ou d'un halogénure de sulfuryle et enfin, dans une troisième étape, on procède à l'acylation des monocarboxylates de monohalogénohydroquinone présents dans le milieu d'halogénation, au moyen d'un agent acylant et éventuellement en présence d'un acide fort.

Comme agent d'acylation des monocarboxylates de monohalogénohydroquinone, on fait appel aux anhydrides d'acides et aux halogénures et de préférence aux chlorures. Les anhydrides sont préférés. Bien que l'on puisse sans sortir du cadre de la présente invention préparer des dicarboxylates mixtes de monohalogénohydroquinone en utilisant un anhydride ou un halogénure d'un acide différent de celui utilisé pour l'obtention du monocarboxylate d'hydroquinone de départ, on préfère mettre en œuvre un anhydride ou un halogénure dérivé du même acide. Comme exemples d'agents acylants on peut citer les anhydrides acétique, propionique et butyrique et les chlorures d'acétyle, de propionyle et de butyryle.

La quantité d'agent acylant est de préférence voisine de la quantité stœchiométrique nécessaire pour acyler les hydroxyles phénoliques présents, c'est-à-dire voisine de 1 mole d'agent acylant par groupe hydroxyle à acyler. On peut cependant sans sortir du cadre de la présente invention utiliser un léger défaut ou un excès d'agent acylant. De préférence la quantité d'agent acylant peut être comprise dans un intervalle de 1 à 5 moles par groupe hydroxyle à acyler. Lorsque le mélange soumis à l'halogénation contient de l'hydroquinone, la quantité d'agent acylant est de préférence suffisante pour acyler totalement l'hydroquinone résiduelle et/ou les halogénohydroquinones présentes dans le milieu d'halogénation en les dicarboxylates d'hydroquinone et/ou les dicarboxylates d'halogénohydroquinone correspondants.

La température de la réaction d'acylation dépend de la nature de l'agent acylant. D'une manière générale elle est comprise entre 0° C et 200° C, et de préférence entre 20° C et 120° C.

Lorsqu'on fait appel à un anhydride d'acide comme agent acylant, on peut opérer en présence ou en absence de tout catalyseur. Lorsqu'on utilise un catalyseur, on fait appel à un acide fort. A cet effet on met en œuvre des acides ayant un pK dans l'eau à 25° C inférieur à 1. On fait appel de préférence à des acides minéraux tels que l'acide sulfurique ou à des acides sulfoniques tels que les acides méthanesulfonique, difluoro- ou trifluorométhanesulfonique, benzènesulfonique, toluènesulfonique ou naphtalènesulfonique. Lorsqu'on fait appel à un mélange de mono et de dicarboxylates provenant d'une réaction de redistribution, le catalyseur acide peut provenir de cette phase. La quantité d'acide fort exprimée en équivalent de protons par groupe hydroxyle à acyler peut être comprise entre $1 \times 10^{-4}$ et $2 \times 10^{-1}$ et de préférence entre $1 \times 10^{-3}$ et $2 \times 10^{-2}$.

La masse réactionnelle résultant de l'acylation est traitée suivant les méthodes usuelles pour séparer les divers constituants du mélange. Lorsque, selon un mode préféré de mise en œuvre de l'invention, on utilise comme matière première de l'étape d'halogénation un mélange de monocarboxylate d'hydroquinone, de dicarboxylate d'hydroquinone et éventuellement d'hydroquinone, la masse réactionnelle résultant de l'acylation contient essentiellement le dicarboxylate de monohalogénohydroquinone résultant de l'acylation du monocarboxylate de monohalogénohydroquinone et éventullement de la monohalogénohydroquinone et le cas échéant une quantité mineure de dicarboxylate de dihalogénohydroquinone et le dicarboxylate d'hydroquinone initialement présent auquel peut s'ajouter celui provenant de l'acylation éventuelle d'hydroquinone résiduelle. Selon un autre mode de réalisation préféré de l'invention, les dicarboxylates d'hydroquinone sont réutilisés pour préparer le mélange initial de monocarboxylate d'hydroquinone, de dicarboxylate d'hydroquinone et éventuellement d'hydroquinone utilisé à la phase d'halogénation, par réaction de redistribution entre un dicarboxylate d'hydroquinone et l'hydroquinone.

Suivant une première variante la masse réactionnelle d'acylation est distillée pour séparer d'abord l'acide solvant, éventuellement l'excès d'agent acylant, le dicarboxylate d'hydroquinone et le dicarboxylate de monohalogénohydroquinone. Le dicarboxylate d'hydroquinone est renvoyé à la zone de redistribution en même temps que l'acide carboxylique solvant. Il suffit d'ajouter des quantités d'hydroquinone et de dicarboxylate d'hydroquinone correspondant à celles consommées au cours de l'halogénation et éventuellement une quantité convenable d'acide fort pour procéder à la préparation du monocarboxylate d'hydroquinone.

Suivant une seconde variante, on ajoute à la masse réactionnelle d'acylation une quantité suffisante d'hydroquinone pour transformer l'agent acylant en excès en dicarboxylate d'hydroquinone puis de procéder à la distillation du mélange réactionnel et de recycler à la phase de redistribution la totalité du dicarboxylate d'hydroquinone et éventuellement l'hydroquinone résiduelle.

Les différentes variantes du procédé selon l'invention conviennent tout particulièrement bien à l'obtention sélective de diacétate de monochlorohydroquinone pratiquement exempt de diacétate de dichlorohydroquinone.

Les exemples suivants illustrent l'invention et montrent comment elle peut être mise en pratique.

*Exemple 1*

Dans un réacteur en verre de 100 ml, équipé d'un agitateur, d'un thermomètre, d'une ampoule de coulée, d'un réfrigérant ascendant et d'un bain de refroidissement, on charge 14,6 g du mélange suivant:

| | | |
|---|---|---|
| – monoacétate d'hydroquinone | 6 g | (0,0395 mole) |
| – diacétate d'hydroquinone | 8,34 g | (0,0430 mole) |
| – hydroquinone | 0,23 g | (0,002 mole) |
| – acide acétique anhydre | 60 ml | |

On introduit goutte à goutte en 30 minutes 5,61 g de chlorure de sulfuryle (0,0416 mole) en maintenant la température à 20° C.

On obtient les résultats suivants, après dosage de la masse réactionnelle par chromatographie liquide sous haute pression (CLHP):

| | |
|---|---|
| – monoacétate d'hydroquinone | 0,00605 mole |
| – monoacétate de monochlorohydroquinone | 0,03475 mole |
| – diacétate d'hydroquinone | 0,0421 mole |

Le taux de transformation du monoacétate d'hydroquinone s'élève à 84,7%.

Le rendement en monoacétate de monochlorohydroquinone par rapport au monoacétate d'hydroquinone transformé s'élève à 100%.

*Exemple 2*

Dans un réacteur de 500 ml équipé comme à l'exemple 1, on charge 55,5 g du mélange suivant:

| | | |
|---|---|---|
| – monoacétate d'hydroquinone | 23,56 g | (0,155 mole) |
| – diacétate d'hydroquinone | 31,04 g | (0,160 mole) |
| – hydroquinone | 0,87 g | (0,0079 mole) |
| – acide acétique | 220 ml | |

On introduit dans le mélange précédent maintenu à 20° C du chlore gazeux à un débit de 5 l/h pendant 54 minutes, soit 4,456 l (0,199 mole).

Après analyse de la masse réactionnelle par CLHP, on obtient un mélange comportant:

| | | |
|---|---|---|
| – monoacétate d'hydroquinone | 5,82 g | (0,0383 mole) |
| – monoacétate de monochlorohydroquinone | 21,82 g | (0,117 mole) |
| – monoacétate de dichloro-2,6 hydroquinone | 0,287 g | (0,0013 mole) |
| – diacétate d'hydroquinone | 27,45 g | (0,1415 mole) |

- diacétate de monochlorohydroquinone 1,76 g (0,0077 mole)
- diacétate de dichloro-2,5 hydroquinone 0,58 g (0,0022 mole)
- diacétate de dichloro-2,3 hydroquinone 0,58 g (0,0022 mole)
- chlorohydroquinone 0,88 g (0,0061 mole)

Le rendement en composés monochlorés (hydroquinone, monoester, diester) par rapport aux réactifs (hydroquinone, monoester et diester) transformés s'élève à 92%.

*Exemple 3*

On opère comme dans l'exemple 2, mais le chlore gazeux est introduit dans la masse réactionnelle maintenue à 80° C. On a dosé les produits suivants dans la masse réactionnelle maintenue à 80° C:

- monoacétate d'hydroquinone 6,795 g (0,0447 mole)
- monoacétate de monochlorohydroquinone 20,68 g (0,1109 mole)
- monoacétate de dichloro-2,6 hydroquinone 0,243 g (0,0011 mole)
- diacétate d'hydroquinone 28,67 g (0,1478 mole)
- diacétate de monochlorohydroquinone 0,594 g (0,0026 mole)
- diacétate de dichloro-2,5 hydroquinone 1,762 g (0,0067 mole)
- diacétate de dichloro-2,3 hydroquinone 0,29 g (0,0011 mole)
- monochlorohydroquinone 0,29 g (0,0020 mole)

Le rendement en composés monochlorés (hydroquinone, monoester, diester) par rapport aux réactifs non chlorés (hydroquinone, monoester et diester) transformés s'élève à 89%.

*Exemple 4*

On opère comme dans l'exemple 2, mais le chlore gazeux est introduit pendant 56 minutes 30 secondes au débit de 5 l/h, soit 4,68 l (0,209 mole) dans la masse réactionnelle maintenue à 20° C.

On a dosé et identifié dans la masse réactionnelle par CLHP les produits suivants:

- monoacétate d'hydroquinone 3,28 g (0,0216 mole)
- monoacétate de monochlorohydroquinone 25,36 g (0,136 mole)
- monoacétate de dichloro-2,6 hydroquinone 0,3 g (0,00135 mole)
- diacétate d'hydroquinone 28,32 g (0,146 mole)
- diacétate de monochlorohydroquinone 1,48 g (0,0065 mole)
- diacétate de dichloro-2,5 hydroquinone 0,89 g (0,0034 mole)
- diacétate de dichloro-2,3 hydroquinone 1,37 g (0,0052 mole)
- monochlorohydroquinone 0,59 g (0,0041 mole)

Le rendement en composés monochlorés (hydroquinone, monoester, diester) par rapport aux réactifs non chlorés (hydroquinone, monoester et diester) transformés s'élève à 94,4%.

*Exemple 5*

Dans un réacteur en verre de 500 ml, muni d'un agitateur, d'un thermomètre, d'un dispositif de chauffage, d'un réfrigérant ascendant et d'une ampoule de coulée, on charge les composés suivants:

- hydroquinone 9,8 g (0,0891 mole)
- diacétate d'hydroquinone 51,8 g (0,267 mole)
- acide acétique anhydre 246 ml
- acide p-toluène-sulfonique 0,64 g

On porte à ébullition le mélange précédent pendant trois heures. On refroidit alors la solution à la température ambiante. On coule ensuite goutte à goutte en 30 minutes environ 22,9 g de chlorure de sulfuryle, soit 0,170 mole en maintenant la température à environ 30° C.

On rajoute 0,1 g d'acide paratoluènesulfonique, puis 91,75 g d'anhydride acétique, soit 0,9 mole, à la solution précédente et on porte le mélange réactionnel à 100° C pendant une nuit.

On a identifié et dosé dans la masse réactionnelle par CLHP les produits suivants:

- diacétate d'hydroquinone 38,8 g (0,2 mole)
- diacétate de monochlorohydroquinone 33,8 g (0,148 mole)
- diacétate de dichloro-2,5 hydroquinone 1,28 g (0,0049 mole)

Le rendement en diacétate de monochlorohydroquinone par rapport à l'hydroquinone et au diacétate d'hydroquinone transformés s'élève à 94,8%.

*Exemple 6*

Dans un réacteur en verre de 500 ml, équipé comme à l'exemple 5, mais dont l'ampoule de coulée a été remplacée par une arrivée de gaz par tube plongeant, on charge les composés suivants:

- hydroquinone 8,92 g (0,0811 mole)
- diacétate d'hydroquinone 47,18 g (0,2432 mole)
- acide acétique anhydre 224 ml
- acide p-toluène-sulfonique 0,58 g

On porte à ébullition le mélange précédent pendant 3 heures. On refroidit alors la solution à la température ambiante. On introduit ensuite en 44 minutes le chlore gazeux à un débit de 5 l/h, soit 3,66 l (0,164 mole) en maintenant la température vers 20° C.

On ajoute 90 g d'anhydride acétique (0,88 mole) et 0,1 g d'acide paratoluènesulfonique et on porte la solution à ébullition pendant 5 heures.

On obtient les résultats suivants après dosage de la masse réactionnelle par CLHP:

– diacétate d'hydroquinone 34,53 g (0,178 mole)
– diacétate de monochlorohydroquinone 32,33 g (0,1415 mole)
– diacétate de dichloro-2,5 hydroquinone 0,75 g (0,00285 mole)

Le rendement en diacétate de monochlorohydroquinone par rapport à l'hydroquinone et au diacétate d'hydroquinone transformés s'élève à 96,7%.

*Exemple 7*

Dans le réacteur en verre de 500 ml, utilisé à l'exemple 6, muni d'un agitateur, on charge les composés suivants:

– hydroquinone 8,92 g (0,0811 mole)
– diacétate d'hydroquinone 47,18 g (0,2432 mole)
– acide acétique anhydre 224 ml
– acide p-toluène-sulfonique 0,52 g

On porte à ébullition le mélange précédent pendant 3 heures. On refroidit légèrement la solution et on introduit ensuite en 44 minutes le chlore gazeux à un débit de 5 l/h, soit 3,66 l (0,164 mole) à la température de 80° C.

Dès la fin de la chloration, on ajoute 90 g d'anhydride acétique (0,88 mole) et 0,1 g d'acide p-toluènesulfonique et on porte la solution à ébullition pendant 5 heures.

On obtient les résultats suivants après dosage de la masse réactionnelle par CLHP:

– diacétate d'hydroquinone (0,194 mole)
– diacétate de monochlorohydroquinone (0,1155 mole)
– diacétate de dichloro-2,5 hydroquinone (0,0098 mole)

Le rendement en diacétate de monochlorohydroquinone par rapport à l'hydroquinone et au diacétate d'hydroquinone transformés s'élève à 88,6%.

*Exemple 8*

On opère comme dans l'exemple 6, mais on introduit à 20° C une quantité plus importante de chlore, soit 3,815 l (0,170 mole).

On obtient les résultats suivants:

– diacétate d'hydroquinone 32,3 g (0,1665 mole)
– diacétate de monochlorohydroquinone 34,05 g (0,149 mole)
– diacétate de dichloro-2,5 hydroquinone 0,89 g (0,0034 mole)

Le rendement en diacétate de monochlorohydroquinone par rapport au diacétate d'hydroquinone et à l'hydroquinone transformés s'élève à 94,4%.

*Exemple 9*

Dans un réacteur de 100 ml équipé d'un agitateur, d'un thermomètre, d'une arrivée de gaz par tube plongeant et d'un bain de refroidissement, on charge:

– hydroquinone 1,56 g (0,0142 mole)
– diacétate d'hydroquinone 8,27 g (0,0426 mole)
– triéthylamine 0,075 g
– éther diisopropylique 15 ml

On chauffe 4 h à 150° C puis on chasse l'éther diisopropylique et la triéthylamine par distillation sous pression réduite. Le résidu est dissous dans 45 ml d'acide acétique anhydre.

Dans la solution maintenue à 20° C, on introduit du chlore gazeux au débit de 5 l/h pendant 6 minutes et 30 secondes, soit 0,54 l (0,024 mole).

Lorsque l'addition du chlore est terminée, on ajoute 10 ml d'anhydride acétique et 0,015 g d'acide paratoluènesulfonique.

On chauffe à 100° C pendant une journée.

Dans la masse réactionnelle finale (poids 68,65 g), on dose par CLHP:

– diacétate d'hydroquinone 6,5 g (0,034 mole)
– diacétate de monochlorohydroquinone 4,95 g (0,022 mole)
– diacétate de 2,5-dichlorohydroquinone 0,2 g (0,0008 mole)

Le rendement en diacétate de monochlorohydroquinone par rapport à l'hydroquinone et au diacétate d'hydroquinone transformés s'élève à 96,5%.

## Revendications

1. Procédé de préparation de monocarboxylate de monohalogénohydroquinone, caractérisé en ce que l'on procède à l'halogénation d'un monocarboxylate d'hydroquinone éventuellement en mélange avec un dicarboxylate d'hydroquinone et une quantité mineure d'hydroquinone au moyen d'un agent halogénant pris dans le groupe formé par le chlore, le brome ou les halogénures de sulfuryle dans un acide carboxylique sensiblement anhydre.

2. Procédé selon la revendication 1, caractérisé en ce que l'on utilise comme milieu de réaction un acide alcanoïque liquide dans les conditions de la réaction et comportant de 1 à 8 atomes de carbone.

3. Procédé selon la revendication 2, caractérisé en ce que l'on utilise l'acide acétique.

4. Procédé selon l'une quelconque des revendications 1 à 3, caractérisé en ce que la quantité d'agent d'halogénation exprimée en moles par mole de monocarboxylates d'hydroquinone est comprise dans un intervalle de 0,5 à 1,5.

5. Procédé selon l'une quelconque des revendications 1 à 4, caractérisé en ce que la température est choisie dans un intervalle de 0 à 120° C.

6. Procédé selon l'une quelconque des revendications 1 à 5, caractérisé en ce que le monocarboxylate d'hydroquinone est utilisé sous forme du

mélange de monocarboxylate d'hydroquinone, de dicarboxylate d'hydroquinone et d'hydroquinone provenant de l'acylation partielle de l'hydroquinone.

7. Procédé selon l'une quelconque des revendications 1 à 5, caractérisé en ce que le monocarboxylate d'hydroquinone est utilisé sous la forme d'un mélange avec un dicarboxylate d'hydroquinone et une quantité mineure d'hydroquinone obtenu par réaction de redistribution d'un dicarboxylate d'hydroquinone avec l'hydroquinone éventuellement en présence d'un catalyseur de transestérification.

8. Procédé selon la revendication 7, caractérisé en ce que la quantité de dicarboxylate d'hydroquinone mise en œuvre au cours de la réaction de redistribution exprimée en moles par mole d'hydroquinone est d'au moins 1,5.

9. Procédé selon l'une quelconque des revendications 7 à 8, caractérisé en ce que la réaction de redistribution est conduite à une température comprise dans un intervalle de 50 à 250° C et à pression normale ou plus élevée.

10. Procédé selon l'une quelconque des revendications 7 à 9, caractérisé en ce que la réaction de redistribution est conduite dans un acide carboxylique liquide dans les conditions de la réaction et sensiblement anhydre éventuellement en présence d'un acide fort.

11. Procédé selon l'une quelconque des revendications 7 à 10, caractérisé en ce que la réaction de redistribution est conduite dans un acide alcanoïque comportant de 1 à 8 atomes de carbone.

12. Procédé selon la revendication 11, caractérisé en ce que la réaction de redistribution est conduite dans l'acide acétique.

13. Procédé selon l'une quelconque des revendications 10 à 11, caractérisé en ce que la réaction de redistribution est conduite en présence d'un acide fort pris dans le groupe des acides sulfurique et organosulfoniques.

14. Procédé selon l'une quelconque des revendications 10 à 11, caractérisé en ce que la quantité d'acide fort exprimée en équivalent de proton par mole d'hydroquinone est comprise dans un intervalle de 0,0001 à 0,2.

15. Procédé selon l'une quelconque des revendications 7 à 9, caractérisé en ce que la réaction de redistribution est conduite dans un éther aliphatique en présence d'un catalyseur pris dans le groupe formé par les bases azotées organiques et les acides de Lewis.

16. Procédé selon la revendication 15, caractérisé en ce que l'on utilise comme catalyseur la triéthylamine.

17. Procédé selon l'une quelconque des revendications 15 à 16, caractérisé en ce que l'on utilise comme éther l'oxyde d'isopropyle.

18. Procédé selon l'une quelconque des revendications 15 à 17, caractérisé en ce que la quantité de catalyseur exprimée en moles par mole d'hydroquinone est comprise dans un intervalle de 0,0001 à 0,2.

19. Procédé de préparation du monoacétate de monochlorohydroquinone, caractérisé en ce que dans une première étape on prépare, par un procédé selon l'une quelconque des revendications 8 à 18, un mélange de diacétate d'hydroquinone et de monoacétate d'hydroquinone contenant une quantité mineure d'hydroquinone par réaction d'au moins 1,5 mole de diacétate d'hydroquinone avec 1 mole d'hydroquinone en solution dans l'acide acétique sensiblement anhydre, éventuellement en présence d'un acide fort, à une température comprise dans un intervalle de 50 à 250° C, puis, dans une deuxième étape, on fait réagir, par un procédé selon l'une quelconque des revendications 1 à 7, le mélange ainsi obtenu avec du chlore gazeux ou du chlorure de sulfuryle à une température comprise entre 0 et 120° C et enfin sépare les produits de la réaction par distillation.

20. Procédé de préparation de dicarboxylates de monohalogénohydroquinone à partir de monocarboxylates de monohalogénohydroquinone obtenus par le procédé de l'une quelconque des revendications 1 à 19, caractérisé en ce que l'on traite les monocarboxylates de monohalogénohydroquinone par un agent d'acylation, à une température comprise dans un intervalle de 0 à 200° C, éventuellement en présence d'un acide fort comme catalyseur, dans un acide carboxylique liquide dans les conditions de la réaction.

21. Procédé selon la revendication 20, caractérisé en ce que l'acylation des monocarboxylates de monohalogénohydroquinone est réalisée directement sur le mélange réactionnel résultant de leur préparation par le procédé selon les revendications 1 à 19.

22. Procédé selon l'une quelconque des revendications 20 à 21, caractérisé en ce que l'on utilise un anhydride d'acide comme agent acylant.

23. Procédé selon l'une quelconque des revendications 20 à 22, caractérisé en ce que la quantité d'agent acylant exprimée en moles par groupe hydroxyle phénolique à acyler est comprise dans un intervalle de 1 à 5.

24. Procédé selon l'une quelconque des revendications 20 à 23, caractérisé en ce que l'on utilise un acide alcanoïque comportant de 1 à 8 atomes de carbone comme milieu réactionnel.

25. Procédé selon l'une quelconque des revendications 20 à 24, caractérisé en ce que l'on utilise comme catalyseur un acide fort pris dans le groupe des acides sulfurique et organosulfoniques.

26. Procédé selon l'une quelconque des revendications 20 à 25, caractérisé en ce que la quantité d'acide fort exprimée en équivalent de proton par groupe hydroxyle phénolique à acyler est comprise dans un intervalle de 0,0001 à 0,2.

27. Procédé selon l'une quelconque des revendications 20 à 26, caractérisé en ce que la réaction d'acylation est conduite à une température comprise dans un intervalle de 20 à 120° C.

28. Procédé selon l'une quelconque des revendications 20 à 27, caractérisé en ce que la réaction d'acylation est conduite sur le mélange réactionnel résultant de l'halogénation d'un mélange de monocarboxylates d'hydroquinone, de dicarboxylates d'hydroquinone et d'hydroquinone selon le procédé des revendications 6 à 19.

29. Procédé de préparation de dicarboxylates de monohalogénohydroquinone selon la revendication 28, caractérisé en ce que le mélange réactionnel résultant de l'acylation est distillé pour séparer ses différents constituants et que le dicarboxylate d'hydroquinone et l'acide carboxylique solvant ainsi récupérés sont réutilisés pour la préparation du monocarboxylate d'hydroquinone par réaction de redistribution avec l'hydroquinone.

30. Procédé selon la revendication 29, caractérisé en ce que, préalablement à la distillation du mélange d'acylation, on ajoute à ce dernier une quantité d'hydroquinone suffisante pour transformer l'éventuel excès d'anhydride d'acide en dicarboxylate d'hydroquinone et monocarboxylate d'hydroquinone.

31. Procédé de préparation du diacétate de monochlorohydroquinone, caractérisé en ce que:

a) dans une première étape on prépare, par un procédé selon l'une quelconque des revendications 8 à 18, un mélange de monoacétate d'hydroquinone, de diacétate d'hydroquinone et d'une quantité mineure d'hydroquinone par réaction de redistribution d'au moins 1,5 mole de dicarboxylate d'hydroquinone avec 1 mole d'hydroquinone en solution dans l'acide acétique sensiblement anhydre éventuellement en présence d'un acide fort à une température comprise dans un intervalle de 50 à 250° C,

b) dans une deuxième étape on fait réagir, par un procédé selon l'une quelconque des revendications 1 à 7, le mélange de la première étape avec un agent d'halogénation pris dans le groupe formé par le chlore et le chlorure de sulfuryle à une température comprise entre 0 et 120° C,

c) dans une troisième étape on procède, selon l'une quelconque des revendications 20 à 28, à l'acétylation des hydroxydes phénoliques libres à l'aide d'anhydride acétique éventuellement en présence d'un acide fort,

d) dans une quatrième étape on procède, selon l'une des revendications 29 ou 30, à la séparation des constituants du mélange réactionnel d'acétylation par distillation, le cas échéant après avoir préalablement transformé l'excès d'anhydride acétique éventuellement présent en diacétate d'hydroquinone et monoacétate d'hydroquinone par réaction avec de l'hydroquinone,

e) dans une cinquième étape on recycle l'acide acétique et le diacétate d'hydroquinone ainsi récupéré à la première phase de redistribution.

## Patentansprüche

1. Verfahren zur Herstellung von Monohalogenhydrochinon-monocarboxylaten, dadurch gekennzeichnet, dass man die Halogenierung eines Hydrochinon-monocarboxylats, gegebenenfalls im Gemisch mit einem Hydrochinon-dicarboxylat und einer geringen Menge Hydrochinon, mittels eines Halogenierungsmittels, genommen aus der Gruppe, gebildet durch Chlor, Brom oder den Sulfurylhalogeniden, in einer im wesentlichen wasserfreien Carbonsäure durchführt.

2. Verfahren gemäss Anspruch 1, dadurch gekennzeichnet, dass man als Reaktionsmilieu eine unter den Reaktionsbedingungen flüssige Alkansäure mit 1 bis 8 Kohlenstoffatomen verwendet.

3. Verfahren gemäss Anspruch 2, dadurch gekennzeichnet, dass man Essigsäure verwendet.

4. Verfahren gemäss einem der Ansprüche 1 bis 3, dadurch gekennzeichnet, dass die Menge an Halogenierungsmittel, ausgedrückt in Mol pro Mol Hydrochinon-monocarboxylate, in einem Bereich von 0,5 bis 1,5 liegt.

5. Verfahren gemäss einem der Ansprüche 1 bis 4, dadurch gekennzeichnet, dass die Temperatur in einem Intervall von 0 bis 120° C ausgewählt ist.

6. Verfahren gemäss einem der Ansprüche 1 bis 5, dadurch gekennzeichnet, dass das Hydrochinon-monocarboxylat in Form eines Gemisches von Hydrochinon-monocarboxylat, Hydrochinon-dicarboxylat und Hydrochinon, stammend von der partiellen Acylierung von Hydrochinon, verwendet wird.

7. Verfahren gemäss einem der Ansprüche 1 bis 5, dadurch gekennzeichnet, dass das Hydrochinon-monocarboxylat in Form eines Gemisches mit einem Hydrochinon-dicarboxylat und einer geringen Menge Hydrochinon verwendet wird, das durch Umverteilungsreaktion eines Hydrochinondicarboxylats mit Hydrochinon, gegebenenfalls in Anwesenheit eines Umesterungskatalysators, erhalten wurde.

8. Verfahren gemäss Anspruch 7, dadurch gekennzeichnet, dass die im Verlauf der Umverteilungsreaktion eingesetzte Menge an Hydrochinon-dicarboxylat, ausgedrückt in Mol pro Mol Hydrochinon, mindestens 1,5 ist.

9. Verfahren gemäss einem der Ansprüche 7 bis 8, dadurch gekennzeichnet, dass die Umverteilungsreaktion bei einer Temperatur in einem Bereich von 50 bis 250° C und bei Normaldruck oder erhöhtem Druck durchgeführt wird.

10. Verfahren gemäss einem der Ansprüche 7 bis 9, dadurch gekennzeichnet, dass die Umverteilungsreaktion in einer unter den Reaktionsbedingungen flüssigen und im wesentlichen wasserfreien Carbonsäure, gegebenenfalls in Gegenwart einer starken Säure, durchgeführt wird.

11. Verfahren gemäss einem der Ansprüche 7 bis 10, dadurch gekennzeichnet, dass die Umverteilungsreaktion in einer Alkansäure mit 1 bis 8 Kohlenstoffatomen durchgeführt wird.

12. Verfahren gemäss Anspruch 11, dadurch gekennzeichnet, dass die Umverteilungsreaktion in Essigsäure durchgeführt wird.

13. Verfahren gemäss einem der Ansprüche 10 bis 11, dadurch gekennzeichnet, dass die Umverteilungsreaktion in Anwesenheit einer starken Säure, ausgewählt aus der Gruppe der Schwefelsäuren und Organosulfonsäuren, durchgeführt wird.

14. Verfahren gemäss einem der Ansprüche 10 bis 11, dadurch gekennzeichnet, dass die Menge an starker Säure, ausgedrückt in Äquivalent Proton pro Mol Hydrochinon, in dem Bereich von 0,0001 bis 0,2 liegt.

15. Verfahren gemäss einem der Ansprüche 7

bis 9, dadurch gekennzeichnet, dass die Umverteilungsreaktion in einem aliphatischen Ether in Gegenwart eines Katalysators, ausgewählt aus der Gruppe, gebildet durch die organischen Stickstoffbasen und die Lewissäuren, durchgeführt wird.

16. Verfahren gemäss Anspruch 15, dadurch gekennzeichnet, dass man als Katalysator Triethylamin verwendet.

17. Verfahren gemäss einem der Ansprüche 15 bis 16, dadurch gekennzeichnet, dass man als Ether Isopropylether verwendet.

18. Verfahren gemäss einem der Ansprüche 15 bis 17, dadurch gekennzeichnet, dass die Menge an Katalysator, ausgedrückt in Mol pro Mol Hydrochinon, in einem Bereich von 0,0001 bis 0,2 liegt.

19. Verfahren zur Herstellung von Monochlorohydrochinon-monoacetat, dadurch gekennzeichnet, dass man in einer ersten Stufe durch ein Verfahren gemäss einem der Ansprüche 8 bis 18 ein Gemisch von Hydrochinon-diacetat und Hydrochinon-monoacetat, das eine geringe Menge Hydrochinon enthält, durch Reaktion von mindestens 1,5 Mol Hydrochinon-diacetat mit 1 Mol Hydrochinon in Lösung in im wesentlichen wasserfreier Essigsäure, gegebenenfalls in Gegenwart einer starken Säure, bei einer Temperatur in dem Bereich von 50 bis 250° C herstellt und dann in einer zweiten Stufe das so erhaltene Gemisch durch ein Verfahren gemäss einem der Ansprüche 1 bis 7 mit gasförmigem Chlor oder Sulfurylchlorid bei einer Temperatur zwischen 0 und 120° C reagieren lässt und schliesslich die Produkte der Reaktion durch Destillation trennt.

20. Verfahren zur Herstellung von Monohalogenhydrochinon-dicarboxylaten, ausgehend von Monohalogenhydrochinon-monocarboxylaten, die erhalten wurden durch das Verfahren gemäss einem der Ansprüche 1 bis 19, dadurch gekennzeichnet, dass man die Monohalogenhydrochinon-monocarboxylate mit einem Acylierungsmittel bei einer Temperatur in dem Bereich von 0 bis 200° C, gegebenenfalls in Anwesenheit einer starken Säure als Katalysator, in einer unter den Reaktionsbedingungen flüssigen Carbonsäure behandelt.

21. Verfahren gemäss Anspruch 20, dadurch gekennzeichnet, dass die Acylierung der Monohalogenhydrochinon-monocarboxylate direkt an dem Reaktionsgemisch durchgeführt wird, das von ihrer Herstellung durch das Verfahren gemäss den Ansprüchen 1 bis 19 resultiert.

22. Verfahren gemäss einem der Ansprüche 20 bis 21, dadurch gekennzeichnet, dass man als Acylierungsmittel ein Säureanhydrid verwendet.

23. Verfahren gemäss einem der Ansprüche 20 bis 22, dadurch gekennzeichnet, dass die Menge an Acylierungsmittel, ausgedrückt in Mol pro zu acylierende, phenolische Hydroxylgruppe, in dem Bereich von 1 bis 5 liegt.

24. Verfahren gemäss einem der Ansprüche 20 bis 23, dadurch gekennzeichnet, dass man eine Alkansäure mit 1 bis 8 Kohlenstoffatomen als Reaktionsmilieu verwendet.

25. Verfahren gemäss einem der Ansprüche 20 bis 24, dadurch gekennzeichnet, dass man als Katalysator eine starke Säure, ausgewählt aus der Gruppe der Schwefelsäuren und Organosulfonsäuren, verwendet.

26. Verfahren gemäss einem der Ansprüche 20 bis 25, dadurch gekennzeichnet, dass die Menge an starker Säure, ausgedrückt in Äquivalent Proton pro zu acylierende, phenolische Hydroxylgruppe, in dem Bereich von 0,0001 bis 0,2 liegt.

27. Verfahren gemäss einem der Ansprüche 20 bis 26, dadurch gekennzeichnet, dass die Acylierungsreaktion bei einer Temperatur in dem Bereich von 20 bis 120° C durchgeführt wird.

28. Verfahren gemäss einem der Ansprüche 20 bis 27, dadurch gekennzeichnet, dass die Acylierungsreaktion an dem Reaktionsgemisch durchgeführt wird, das von der Halogenierung eines Gemisches von Hydrochinon-monocarboxylaten, Hydrochinon-dicarboxylaten und Hydrochinon gemäss dem Verfahren der Ansprüche 6 bis 19 resultiert.

29. Verfahren zur Herstellung von Monohalogenhydrochinon-dicarboxylaten gemäss Anspruch 28, dadurch gekennzeichnet, dass das von der Acylierung resultierende Reaktionsgemisch destilliert wird, um seine verschiedenen Bestandteile zu trennen, und dass das so wiedergewonnene Hydrochinon-dicarboxylat und Carbonsäure-Lösungsmittel wiederverwendet werden zur Herstellung des Hydrochinon-monocarboxylats durch Umverteilungsreaktion mit dem Hydrochinon.

30. Verfahren gemäss Anspruch 29, dadurch gekennzeichnet, dass vor der Destillation des Acylierungsgemisches zu dem letzteren eine ausreichende Menge Hydrochinon zugesetzt wird, um den eventuellen Überschuss an Säureanhydrid in Hydrochinon-dicarboxylat und Hydrochinon-monocarboxylat zu überführen.

31. Verfahren zur Herstellung von Monochlorohydrochinon-diacetat, dadurch gekennzeichnet, dass man:

(a) in einer ersten Stufe durch ein Verfahren gemäss einem der Ansprüche 8 bis 18 ein Gemisch von Hydrochinon-monoacetat, Hydrochinon-diacetat und einer geringen Menge Hydrochinon durch Umverteilungsreaktion von mindestens 1,5 Mol Hydrochinon-dicarboxylat mit 1 Mol Hydrochinon in Lösung in im wesentlichen wasserfreier Essigsäure und gegebenenfalls in Anwesenheit einer starken Säure bei einer Temperatur in dem Bereich von 50 bis 250° C herstellt,

(b) in einer zweiten Stufe das Reaktionsgemisch der ersten Stufe durch ein Verfahren gemäss einem der Ansprüche 1 bis 7 mit einem Halogenierungsmittel, ausgewählt aus der Gruppe, gebildet durch Chlor und Sulfurylchlorid, bei einer Temperatur zwischen 0 und 120° C umsetzt,

(c) in einer dritten Stufe gemäss einem der Ansprüche 20 bis 28 die Acetylierung der freien phenolischen Hydroxyle mittels Essigsäureanhydrid, gegebenenfalls in Gegenwart einer starken Säure, durchführt,

(d) in einer vierten Stufe gemäss einem der Ansprüche 29 oder 30 die Trennung der Bestandteile des Acetylierungsreaktionsgemisches durch Destillation durchführt, gegebenenfalls nachdem vorher der Überschuss an gegebenenfalls vorhandenem Essigsäureanhydrid zu Hydrochinon-diacetat und Hydrochinon-monoacetat durch Reaktion mit dem Hydrochinon überführt wurde,

(e) in einer fünften Stufe die Essigsäure und das so wiedergewonnene Hydrochinon-diacetat zu der ersten Umverteilungsphase zurückführt.

## Claims

1. Process for the preparation of monohalohydroquinone monocarboxylates, characterized in that the halogenation of a hydroquinone monocarboxylate, optionally mixed with a hydroquinone dicarboxylate and a small quantity of hydroquinone, is carried out using a halogenating agent chosen from the group consisting of chlorine, bromine or sulphuryl halides in a substantially anhydrous carboxylic acid.

2. Process according to Claim 1, characterized in that an alkanoic acid which is liquid under the reaction conditions and contains 1 to 8 carbon atoms, is employed as the reaction medium.

3. Process according to Claim 2, characterized in that acetic acid is employed.

4. Process according to any one of Claims 1 to 3, characterized in that the quantity of halogenating agent expressed in moles per mole of hydroquinone monocarboxylate is within a range from 0.5 to 1.5.

5. Process according to any one of Claims 1 to 4, characterized in that the temperature is chosen within a range from 0 to 120° C.

6. Process according to any one of Claims 1 to 5, characterized in that the hydroquinone monocarboxylate is employed in the form of a mixture of hydroquinone monocarboxylate, hydroquinone dicarboxylate and hydroquinone, originating from the partial acylation of hydroquinone.

7. Process according to any one of Claims 1 to 5, characterized in that the hydroquinone monocarboxylate is employed in the form of a mixture with a hydroquinone dicarboxylate and a small quantity of hydroquinone obtained by the redistribution reaction of a hydroquinone dicarboxylate with hydroquinone, optionally in the presence of a transesterification catalyst.

8. Process according to Claim 7, characterized in that the quantity of hydroquinone dicarboxylate employed during the redistribution reaction, expressed in moles per mole of hydroquinone, is at least 1.5.

9. Process according to either of Claims 7 and 8, characterized in that the redistribution reaction is carried out at a temperature within a range from 50 to 250° C and at normal or higher pressure.

10. Process according to any one of Claims 7 to 9, characterized in that the redistribution reaction is carried out in a carboxylic acid which is liquid under the reaction conditions and substantially anhydrous, optionally in the presence of a strong acid.

11. Process according to any one of Claims 7 to 10, characterized in that the redistribution reaction is carried out in an alkanoic acid containing from 1 to 8 carbon atoms.

12. Process according to Claim 11, characterized in that the redistribution reaction is carried out in acetic acid.

13. Process according to either of Claims 10 and 11, characterized in that the redistribution reaction is carried out in the presence of a strong acid chosen from the group consisting of sulphuric and organosulphonic acids.

14. Process according to either of Claims 10 and 11, characterized in that the quantity of strong acid, expressed in equivalents of protons per mole of hydroquinone, is within a range from 0.0001 to 0.2.

15. Process according to any one of Claims 7 to 9, characterized in that the redistribution reaction is carried out in an aliphatic ether in the presence of a catalyst chosen from the group consisting of organic nitrogenous bases and Lewis acids.

16. Process according to Claim 15, characterized in that triethylamine is employed as catalyst.

17. Process according to either of Claims 15 and 16, characterized in that isopropyl ether is employed as the ether.

18. Process according to any one of Claims 15 to 17, characterized in that the quantity of catalyst, expressed in moles per mole of hydroquinone, is within a range from 0.0001 to 0.2.

19. Process for the preparation of monochlorohydroquinone monoacetate, characterized in that, in a first stage, a mixture of hydroquinone diacetate and hydroquinone monoacetate containing a small quantity of hydroquinone is prepared, by a process according to any one of Claims 8 to 18, by reacting at least 1.5 mole of hydroquinone diacetate with 1 mole of hydroquinone dissolved in substantially anhydrous acetic acid, optionally in the presence of a strong acid, at a temperature within a range from 50 to 250° C and then, in a second stage, the mixture thereby obtained is reacted, by a process according to any one of Claims 1 to 7, with gaseous chlorine or sulphuryl chloride at a temperature between 0 and 120° C and the reaction products are finally separated by distillation.

20. Process for the preparation of monohalohydroquinone dicarboxylates from monohalohydroquinone monocarboxylates obtained by the process of any one of Claims 1 to 19, characterized in that the monohalohydroquinone monocarboxylates are treated with an acylating agent, at a temperature within a range from 0 to 200° C, optionally in the presence of a strong acid as catalyst, in a carboxylic acid which is liquid under the reaction conditions.

21. Process according to Claim 20, characterized in that the acylation of the monohalohydroquinone monocarboxylates is carried out directly on the reaction mixture resulting from the preparation thereof by the process according to Claims 1 to 19.

22. Process according to either of Claims 20 and 21, characterized in that an acid anhydride is employed as acylating agent.

23. Process according to any one of Claims 20 to 22, characterized in that the quantity of acylating agent, expressed in moles per phenolic hydroxyl group to be acylated, is within a range from 1 to 5.

24. Process according to any one of Claims 20 to 23, characterized in that an alkanoic acid containing 1 to 8 carbon atoms is employed as reaction medium.

25. Process according to any one of Claims 20 to 24, characterized in that a strong acid chosen from the group consisting of sulphuric and organosulphonic acids is employed as catalyst.

26. Process according to any one of Claims 20 to 25, characterized in that the quantity of strong acid, expressed as equivalents of protons per phenolic hydroxyl group to be acylated, is within a range from 0.0001 to 0.2.

27. Process according to any one of Claims 20 to 26, characterized in that the acylation reaction is carried out at a temperature within a range from 20 to 120° C.

28. Process according to any one of Claims 20 to 27, characterized in that the acylation reaction is carried out on the reaction mixture resulting from the halogenation of a mixture of hydroquinone monocarboxylates, hydroquinone dicarboxylates and hydroquinone according to the process of Claims 6 to 19.

29. Process for the preparation of monohalohydroquinone dicarboxylates according to Claim 28, characterized in that the reaction mixture resulting from the acylation is distilled in order to separate the different constituents thereof and in that the hydroquinone dicarboxylate and the solvent carboxylic acid thereby recovered are reused for the preparation of hydroquinone monocarboxylate by the redistribution reaction with hydroquinone.

30. Process according to Claim 29, characterized in that prior to the distillation of the acylation mixture, a quantity of hydroquinone sufficient to convert any excess acid anhydride into hydroquinone dicarboxylate and hydroquinone monocarboxylate is added to the latter.

31. Process for the preparation of monochlorohydroquinone diacetate characterized in that:

a) in a first stage, a mixture of hydroquinone monoacetate, hydroquinone diacetate and a small quantity of hydroquinone is prepared, by a process according to any one of Claims 8 to 18, by the redistribution reaction of at least 1.5 mole of hydroquinone dicarboxylate with 1 mole of hydroquinone dissolved in substantially anhydrous acetic acid, optionally in the presence of a strong acid, at a temperature within a range from 50 to 250° C,

b) in a second stage, the mixture from the first stage is reacted, by a process according to any one of Claims 1 to 7, with a halogenating agent chosen from the group consisting of chlorine and sulphuryl chloride, at a temperature between 0 and 120° C,

c) in a third stage, the acetylation of the free phenolic hydroxyl groups is carried out, according to any one of Claims 20 to 28, using acetic anhydride, optionally in the presence of a strong acid,

d) in a fourth stage, the separation of the constituents of the acetylation reaction mixture is carried out, according to one of Claims 29 or 30, by distillation, after converting beforehand, where appropriate, the excess acetic anhydride, if present, into hydroquinone diacetate and hydroquinone monoacetate by reacting with hydroquinone,

e) in a fifth stage, the acetic acid and the hydroquinone diacetate thus recovered in the first redistribution phase is recycled.